# EUROPÄISCHE PATENTANMELDUNG

(11) **EP 2 363 402 A1**
(43) Veröffentlichungstag der Anmeldung: **07.09.2011**
(21) Anmeldenummer: 10155095.2
(22) Anmeldetag: 01.03.2010
(51) Int. Cl.: C07F 15/00, C07F 19/00, B01J 31/24, C07C 45/50, C07C 47/02, C07F 9/50

(54) **Polyhedrale Oligomere Silsesquioxan (POSS)-verbundene Liganden und deren Verwendung**

(71) Anmelder: Evonik Oxeno GmbH, 45772 Marl (DE)
(72) Erfinder: Christiansen, Andrea Dr., 45657 Recklinghausen (DE); Franke, Robert Dr., 45772 Marl (DE); Hess, Dieter Dr., 45770 Marl (DE); Fridag, Dirk, 45721 Haltern am See (DE); Vogt, Dieter Prof., 5672 HN Nuenen (NL); Müller, Christian Dr., 5612EJ Eindhoven (NL); Janssen, Michèle, 5645 JD Eindhoven (NL)

(57) **Zusammenfassung**

Gegenstand der vorliegenden Erfindung sind POSS-modifizierte Liganden und deren Verwendung in katalytisch wirksamen Zusammensetzungen in der Hydroformylierung.

## Beschreibung

Die Hydroformylierung von Olefinen und olefinhaltigen Gemischen sind ein Forschungsgegenstand der chemischen Industrie. Eine dauerhafte Problemstellung ist bei der katalytischen Hydroformylierung der Erhalt der Aktivität und der Selektivität der jeweils verwendeten katalytisch wirksamen Zusammensetzungen gegenüber den zu hydroformylierenden Olefinen und olefinhaltigen Gemischen unter den Reaktionsbedingungen. Insbesondere bei Übergangsmetallhaltigen, katalytisch wirksamen Zusammensetzungen ist es ein Ziel der Forschung die Inhibierung der katalytischen Wirkung, die Bildung von Übergangsmetall-Clustern und den Ausfall des Übergangsmetalls selbst zu verhindern, zumindest deutlich einzuschränken. Die vorliegende Erfindung liefert einen Beitrag zu dieser Problemstellung, indem sie einen Weg aufzeigt, wie unter Vermeidung von thermischer Belastung des Reaktionsgemisches auf einfache Weise die gewünschten Zielprodukte abgetrennt werden von der katalytisch wirksamen Zusammensetzung, wobei dessen katalytische Aktivität erhalten bleibt.

Ein Gegenstand der vorliegenden Erfindung sind POSS-modifizierte Liganden, wobei unter POSS Polyhedrale Oligomere Silsesquioxan-Derivate verstanden werden. Die verwendeten Polyhedralen Oligomeren Silsesquioxan-Derivate werden mit an sich bekannten Ligandenvorstufen umgesetzt. Die daraus resultierenden POSS-modifizierten Liganden weisen ein drastisch erhöhtes Molekulargewicht im Vergleich zu unmodifizierten Liganden auf. In einer Ausführungsform der Erfindung werden auf Basis von Triorganophosphinen, wie Triphenylphosphin beispielsweise Alkylphenyl-substituierte, insbesondere ein Ethylphenyl-substituiertes, mit POSS substituiertes Triphenylphosphinderivat hergestellt:

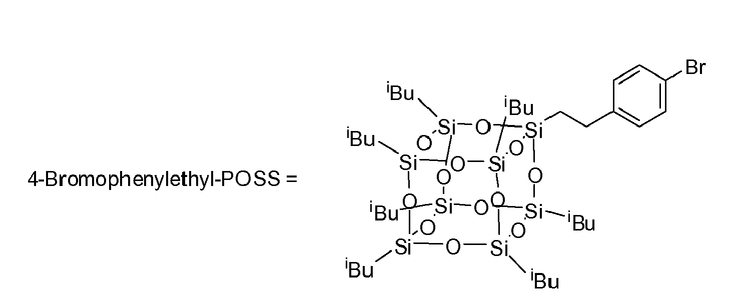

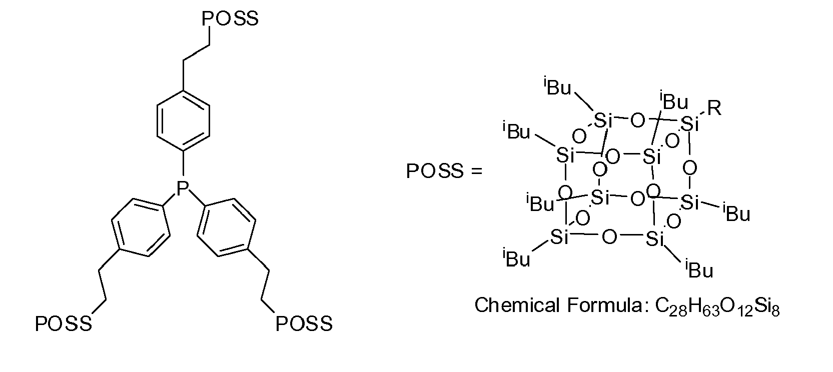

### Herstellung von POSS-substituierten Triphenylphosphin

4-Bromophenylethyl-POSS (11 g, 10.92 mmol) wird gelöst in 100 mL THF und auf -78°C gekühlt. n-BuLi (2.5 M in hexanes, 4.8 mL, 12 mmol) wird tropfenweise zugefügt und die Reaktionsmischung wird bei dieser Temperatur 1 h gerührt. PCl3 (0.5 g, 3.64 mmol) wird tropfenweise hinzugegeben. Man lässt die Reaktionsmischung auf Raumtemperatur erwärmen und rührt über Nacht. Das Lösungsmittel wird im Vakuum entfernt. Das Reaktionsprodukt wird aus dem verbleibenden Feststoff mit Toluol/Hexan (1:1, 150 mL) extrahiert und mit entgastem Wasser gewaschen. Die organische Phase wird über MgSO₄ getrocknet. Die Lösungsmittel werden im Vakuum entfernt. Das Produkt wird erhalten als ein weißer Feststoff in 78% Ausbeute (8 g, 2.84 mmol).
1H NMR δ (ppm): 7.24 (dd, J = 12.4 Hz, J = 14.4 Hz, 12H), 2.69 (m. 6H), 1.87 (sept, J = 6.7 Hz, 7H), 0.97 (d, J = 6.6 Hz, 42H), 0.62 (d, J = 7 Hz, 14H)
13C NMR δ (ppm): 145.15, 133.83, 127.94, 125.61, 28.97, 25.70, 23.88, 22.53, 14.07
31 P NMR ö (ppm): - 7.76 (s)
Maldi-Tof: m/z = 2809.07 (M+Na)

**Elementaranalyse:** berechnet (gefunden): C 46.45 (46.54), H 7.69 (7.77)

Ein weiterer Gegenstand der vorliegenden Erfindung sind in der Hydroformylierung von Olefinen und olefinhaltigen Gemischen katalytisch wirksame, Übergangsmetallhaltige Zusammensetzungen, die durch Umsetzung der POSS-modifizierten Liganden mit geeigneten Übergangsmetallvorstufen erhältlich sind. Kennzeichnend für diese neuartigen, mit POSS-modifizierten Liganden hergestellten Übergangsmetallkomplexe in den katalytisch wirksamen Zusammensetzungen ist, dass die Aktivität sowie Selektivität gegenüber den nicht mit POSS-modifizierten Übergangsmetallkomplexen erhalten bleibt. Zugleich sind die erfindungsgemäßen, katalytisch wirksamen Zusammensetzungen vollständig aus dem Reaktionsgemisch mittels organischer Nanofiltration abtrennbar und können in die Hydroformylierungsreaktion zurückgeführt werden. In einer Ausführungsform der Erfindung wird das zuvor beschriebene POSS-substituierte Triphenylphoshin mit einer Rhodiumhaltigen, geeigneten Übergangsmetallvorstufe, wie z. B. [Rh(acac)(CO)₂], zu der katalytisch wirksamen Zusammensetzung umgesetzt.

Ein weiterer Gegenstand der vorliegenden Erfindung ist die Verwendung von POSS-modifizierten Liganden in katalytisch wirksamen Zusammensetzungen in der Hydroformylierung von Olefinen und olefinhaltigen Gemischen. In einer Ausführungsform der Erfindung wird das zuvor beschriebene POSS-substituierte Triphenylphosphin mit einer Rhodiumhaltigen, geeigneten Übergangsmetallvorstufe, wie z.B. [Rh(acac)(CO)₂], zu der katalytisch wirksamen Zusammensetzung umgesetzt, welche in der Hydroformylierung von Olefinen, wie z. B. 1-Okten, eingesetzt wird:

### Hydroformylierung von 1-Okten in einem kontinuierlich betriebenen Membranreaktor

Die Hydroformylierungsversuche wurden durchgeführt in einem 100 mL Parr Autoklaven ausgestattet mit Druckregulierung, Gasflussmessung, Tropftrichter und Rührer. Vor dem Start wurde der Reaktor 5-mal mit 20 bar Synthesegas CO/H₂ (1:1) gespült. Unter einem leichten Argonstrom wurde die Substratlösung (1.92 M 1-OKtene in Toluol) mittels HPLC Pumpe in den Reaktor überführt. Der Reaktor wurde auf 80°C erhitzt und ein Druck von 20 bar CO/H₂ (1:1) eingestellt. Das Reaktionssystem wurde über 1h äquilibriert, bevor die katalytisch wirksame Zusammensetzung, enthaltend 15 mg (58 µmol) Rh(acac)(CO)₂ und 815 mg (290 µmol) des POSS-substituierten PPh₃ in 14 mL Toluol, hinzugegeben wurde via Tropftrichter (t =0). Die Reaktion wurde über einen Zeitraum von 14 Tagen durchgeführt, währenddessen Proben gezogen wurden in regelmäßigen Intervallen. Der Umsatz von 1-Okten und die Regioselektivität (l/b ratio) wurden mittels GC Analyse bestimmt.

Kontinuierliche Hydroformylierung von 1-Okten mit POSS-substituierten PPh₃ /Rh

| Probe | Zeit | Umsatz (%) | Ausbeute | l/b |
|---|---|---|---|---|
| | (min) | | (%) | |
| 1 | 0 | 0.0 | 0 | |
| 2 | 10 | 0.0 | 0.0 | |
| 3 | 20 | 1.3 | 1.3 | |
| 4 | 30 | 4.0 | 4.0 | 2.8 |
| 5 | 40 | 6.9 | 6.9 | 2.8 |
| 6 | 50 | 10.3 | 10.3 | 2.8 |
| 7 | 60 | 13.6 | 13.6 | 2.8 |
| 8 | 70 | 17.3 | 17.3 | 2.8 |
| 9 | 80 | 20.8 | 20.8 | 2.8 |
| 10 | 90 | 24.2 | 23.5 | 2.8 |
| 11 | 100 | 28.2 | 27.4 | 2.8 |
| 12 | 110 | 31.4 | 30.5 | 2.8 |
| 13 | 120 | 35.1 | 34.2 | 2.8 |
| 14 | 140 | 41.9 | 40.7 | 2.8 |
| 15 | 160 | 48.1 | 46.8 | 2.8 |
| 16 | 180 | 53.6 | 52.1 | 2.8 |
| 17 | 240 | 69.8 | 67.1 | 2.8 |
| 18 | 300 | 83.3 | 79.9 | 2.8 |
| 19 | 360 | 93.4 | 89.7 | 2.8 |
| 20 | 420 | 97.1 | 93.4 | 2.8 |
| 21 | 1020 | 98.6 | 95.8 | 2.5 |
| 22 | 1080 | 98.6 | 95.7 | 2.5 |
| 23 | 1140 | 98.6 | 95.7 | 2.5 |
| 24 | 1200 | 98.6 | 95.7 | 2.5 |
| 25 | 1260 | 98.7 | 95.7 | 2.5 |
| 26 | 1620 | 98.7 | 96.0 | 2.5 |
| 27 | 2490 | 98.8 | 96.3 | 2.4 |
| 28 | 3030 | 98.7 | 96.1 | 2.3 |
| 29 | 3930 | 98.7 | 96.2 | 2.3 |
| 30 | 5650 | 98.6 | 96.5 | 2.3 |
| 31 | 7050 | 98.6 | 96.5 | 2.3 |

In weiteren Ausführungsformen der Erfindung zu der Verwendung von POSS-modifizierten Liganden in katalytisch wirksamen Zusammensetzungen in der Hydroformylierung werden als olefinhaltige Gemische u. a. Raffinate, wie z. B. Raffinat I, Raffinat II, aber auch Mischungen, die Olefine mit 3 bis 20 Kohlenstoffatomen enthalten, eingesetzt.

## Patentansprüche

1. Organische Phosphorhaltige Verbindung, kovalent verbunden mit Polyhedralen
Oligomeren Silsesquioxanderivaten gemäß Formel 1 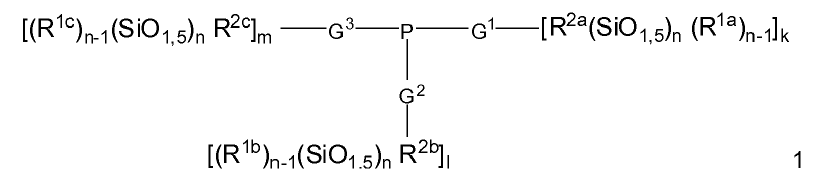 wobei (R^{1a,b,c})ₙ₋₁(SiO₁,₅)ₙR^{2a,b,c} Polyhedrale Oligomere Silsesquioxanderivate darstellen mit n ≥ 4;
in denen R^{1a}, R^{1b},R^{1c} jeweils unabhängig ausgewählt sind aus der Gruppe bestehend aus gleichen oder unterschiedlichen, verzweigten oder linearen C₁-C₂₀ Alkylketten, Cycloalkyl-, C₁-C₂₀ Alkoxy-, Aryl-, Aryloxy-, Heteroaryl- und Arylalkylgruppen, worin k, l, m 0 oder 1 ist, unter der Voraussetzung, dass k+l+m ≥ 1,
in denen R^{2a}, R^{2b}, R^{2c} die Verknüpfung zwischen Polyhedralen Oligomeren Silsesquioxanderivaten zu G1, G2 und/oder G3 darstellen,
wobei R^{2a}, R^{2b}, R^{2c} jeweils unabhängig voneinander ausgewählt sind aus der Gruppe bestehend aus linearen oder verzweigten C₁-C₂₀Alkylketten, C₃-C₁₀ Cycloalkyl-, C₁-C₂₀ Alkoxy-, C₂-C₂₀ Alkenyl-, C₂-C₂₀ Alkenyloxy-, Aryloxy-, C₁-C₂₀ Alkylthio-, C₁-C₂₀ Carboxylat-, Aryl or Heteroaryl-, C₁-C₂₀ Alkylhalogenid-, anellierten Aryl- oder Heteroaryl-, C₃-C₁₀ Cycloalkylgruppen, Ethern, Polyethern, Polythioethern, Aminen, Amiden, Carboxylaten, Aryl-verbrückten Alkylketten, in denen die Arylstruktur weitere Substituenten aufweisen kann;
wobei G1, G2 und G3 jeweils gleiche oder verschiedene monovalent mit Phosphor verbundene Einheiten darstellen, ausgewählt aus der Gruppe bestehend aus unsubstituierten und/oder substituierten aliphatischen, cycloaliphatischen, heterocycloaliphatischen, perfluoralkylierten, aromatischen, heteroaromatischen, kondensierten aromatischen, kondensierten heteroaromatischen Einheiten.

2. Organische Phosphorhaltige Verbindung nach Anspruch 1,
wobei n die Werte von 6 bis 12 annimmt,
in denen R^{1a}, R^{1b}, R^{1c} jeweils gleich und C₁-C₈ Alkylketten, oder Phenylgruppen sind, und worin G1, G2 und/oder G3 jeweils gleiche oder verschiedene monovalent mit Phosphor verbundene Einheiten darstellen, ausgewählt aus der Gruppe Acyl, Alkyl, Aryl, Heteroaryl, Cycloalkyl.

3. Organische Phosphorhaltige Verbindung nach Anspruch 2, wobei n = 8 ist und R^{1a}, R^{1b}, R^{1c} jeweils gleich und C₄ Alkylketten sind,
worin R^{2a}, R^{2b}, R^{2c} jeweils gleich und C₂-Alkylketten sind,
wobei G1, G2 und G3 gleich und jeweils monovalent mit Phosphor verknüpfte Phenylgruppen sind.

4. Katalytisch wirksame Zusammensetzung, enthaltend mindestens eine organische Phosphorhaltige Verbindung gemäß der Ansprüche 1 - 3 und mindestens ein Metall, ausgewählt aus der 8., 9. oder 10. Gruppe des Periodensystems der Elemente.

5. Katalytisch wirksame Zusammensetzung nach Anspruch 4, **dadurch gekennzeichnet, dass** das Metall ausgewählt ist aus der 9. Gruppe des Periodensystems der Elemente.

6. Katalytisch wirksame Zusammensetzung nach Anspruch 5, **dadurch gekennzeichnet, dass** das Metall Rhodium ist.

7. Verfahren zur Hydroformylierung von olefinhaltigen Gemischen, enthaltend eine katalytisch wirksame Mischung gemäß der Ansprüche 4 - 6.

8. Verfahren zur Hydroformylierung von olefinhaltigen Gemischen nach Anspruch 7,
**dadurch gekennzeichnet, dass** das olefinhaltige Gemisch Olefine mit 3 bis 20 Kohlenstoffatomen enthält.

9. Verfahren zur Hydroformylierung von olefinhaltigen Gemischen nach Anspruch 8,
**dadurch gekennzeichnet, dass** das olefinhaltige Gemisch ausgewählt ist aus der Gruppe bestehend aus Propen, Raffinat I, Rafffinat II, Raffinat III.

10. Verfahren zur Hydroformylierung von olefinhaltigen Gemischen nach Anspruch 8,
**dadurch gekennzeichnet, dass** das olefinhaltige Gemisch 1-Okten enthält.

11. Verfahren zur Hydroformylierung von olefinhaltigen Gemischen nach den Ansprüchen 7 - 10, **dadurch gekennzeichnet, dass** die katalytisch wirksame Zusammensetzung unter Verzicht auf thermische Trennverfahren mittels organischer Nanofiltration von einem das Produkt enthaltenden Strom abgetrennt wird.
